# EUROPEAN PATENT APPLICATION

(11) **EP 4 631 679 A1**
(43) Date of publication of application: **15.10.2025**
(21) Application number: 25167036.0
(22) Date of filing: 28.03.2025
(51) Int. Cl.: B25J 11/00, B25J 15/00, A61F 2/30, A61F 2/38

(54) **GRIPPING SUPPORT AND ROBOTIC STATION FOR FINISHING A SEMI-FINISHED PIECE, IN PARTICULAR A FEMORAL COMPONENT OF A KNEE PROSTHESIS**

(30) Priority: 11.04.2024 IT 202400008131
(71) Applicant: Robot Service S.r.l., 25013 Carpenedolo, Brescia (IT)
(72) Inventor: ZANINELLI, Devis, I-25013 Carpenedolo, BRESCIA (IT)
(74) Representative: Pulieri, Gianluca Antonio

(57) **Abstract**

A gripping support (200) for a semi-finished product (S1, S3) of the femoral component (1) of a knee prosthesis comprises adjustable engagement means (220) for releasably engaging semi-finished products of different sizes. The engagement means (220) comprise releasable forcing means adapted to apply a locking action to the pins (8a,8b) of the semi-finished product. A robotic station (100) to perform de-burring and grinding processes on semi-finished chrome-cobalt pieces (S1, S3) of femoral knee prosthesis components (1) comprises a de-burring apparatus (116), a grinding apparatus (136) and at least one anthropomorphic robot (114, 134).

## Description

### Field of the invention

The present invention is in the field of equipment and machinery for carrying out finishing processes such as the de-burring, grinding, and polishing of semi-finished metallic pieces. In particular, the present invention relates to a gripping support and a robotic station for de-burring and grinding the femoral component of a knee prosthesis.

### Prior art

A knee prosthesis usually consists of a femoral component, which is attached to the end of the femur, and a tibial component, which is attached to the tibia. The two components, which in operation push one on top of the other, are articulated therebetween and separated by a disk made of a plastic material. The two components are each made as one piece from a chromium-cobalt alloy.

The production of such components, which have a rather complex shape, is particularly difficult insofar as very tight processing tolerances, high surface finishes and stringent constraints are required. This is also the case, in order to prevent components from being polluted by substances that may compromise the biological compatibility thereof, for the equipment that is used to perform these various processes.

### Object of the invention

The object of the present invention is to provide a gripping support for the femoral component and a robotic station that meet the needs of the industry and overcome the drawbacks of the solutions of the prior art, with particular reference to the timing and quality of the finishing processes.

Such object is achieved by a gripping support according to claim 1 and a robotic station according to claim 10. The dependent claims describe additional advantageous embodiments of the invention.

### Brief description of the figures

The features and advantages of the gripping support and robotic station according to the present invention will be apparent from the description below, given by way of non-limiting example in accordance with the figures in the attached drawings, wherein:
- Figures 1 and 2 show a femoral component of a knee prosthesis;
- Figure 2 represents a robotic station according to one embodiment of the present invention;
- Figure 4 depicts a feeding device packed with gripping supports carrying a semi-finished product of the femoral component to be de-burred;
- Figure 5 depicts a storage device packed with gripping supports carrying a ground semi-finished product of the femoral component;
- Figure 6 shows a gripping support according to one embodiment of the present invention;

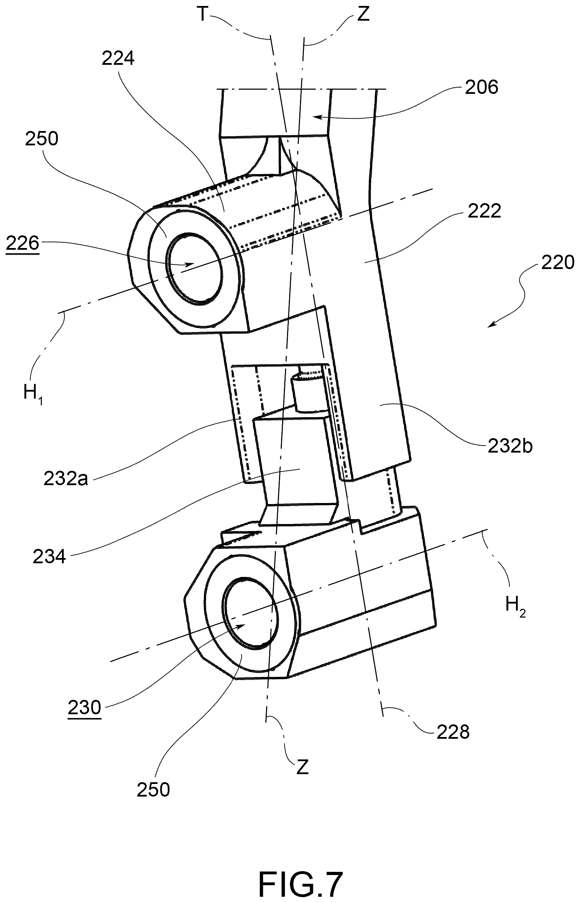

- Figure 7 shows engagement means of the gripping device;

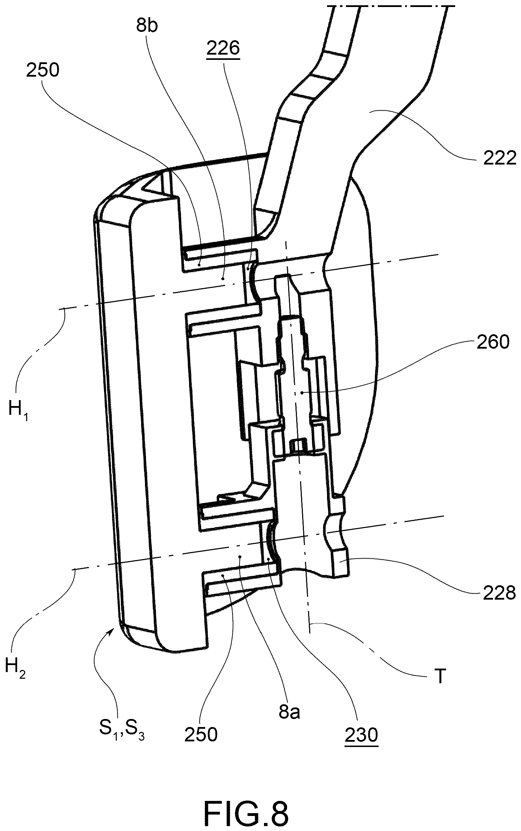

- Figure 8 depicts a cross-section of the engagement means.

### Detailed description of the invention

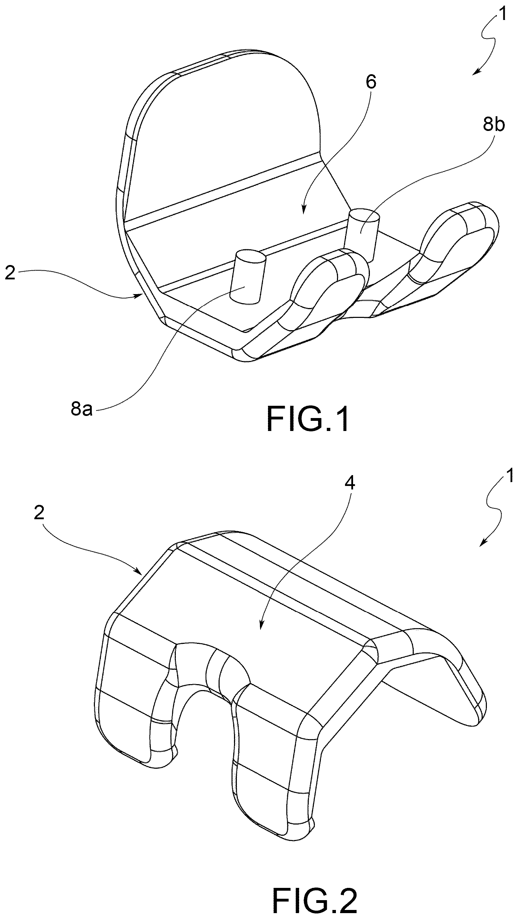

With reference to Figures 1 and 2, a knee prosthesis comprises a femoral component 1 intended to be attached to the end of the femur of a patient. The femoral component 1 consists of a single piece made of a metallic material, preferably a chromium-cobalt alloy. The semi-finished product is obtained by casting, it is then subjected to de-burring and grinding processing on the robotic station, object of the present invention.

The femoral component 1 comprises a cap 2, delimited by a curved outer surface 4 and a curved inner surface 6, and a pair of pins 8a, 8b, protruding from the inner surface 6. The pins 8a, 8b are used for fixing the femoral component 1 to the femur.

In order to adapt perfectly to the bone characteristics of the patient, knee prostheses have different sizes; from smaller sizes to larger ones; the dimensions of the components generally increase. In particular, as regards the femoral component, from the smallest to the largest sizes the distance between the two pins 8a, 8b increases.

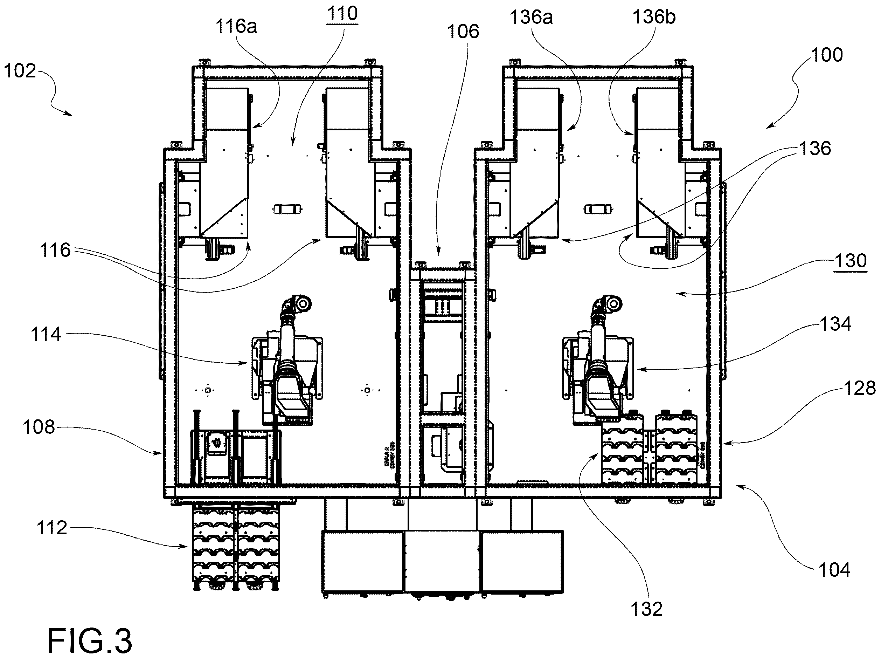

According to Figure 3, a robotic station 100 according to one embodiment of the present invention, adapted to carry out a de-burring and a grinding process of a semi-finished product from which to obtain the femoral component 1, preferably comprises a de-burring assembly 102 and a grinding assembly 104, preferably side by side and functionally correlated by means of a transfer assembly 106 arranged between them.

The de-burring assembly 102 comprises a peripheral wall 108 that delimits an inner region 110, accessible for example from a side door.

The de-burring assembly 102 further comprises a feed device 112, operating for example through the peripheral wall 108, adapted to be moved in order to transport a plurality of semi-finished products to be de-burred S1 into the region 110. For example, the feed device 112 is a translatable tray.

The de-burring assembly 102 further comprises an anthropomorphic robot 114, located within the region 110, in proximity to the feed device 112 for collecting the semi-finished products to be de-burred S1.

The de-burring assembly 102 further comprises a de-burring apparatus 116 adapted to perform de-burring processing on the semi-finished products to be de-burred S1, located within the region 110, so that the robot 114 may approach the semi-finished products to be de-burred S1 in order to perform the de-burring processing.

For example, the de-burring apparatus 116 comprises a first de-burring device 116a and a second de-burring device 116b, arranged in front of the robot 114, transversely separated one from the other, each comprising one or more de-burring wheels whereupon finishing strips are applied that are adapted to perform de-burring processes on metal semi-finished products, in particular made of chromium-cobalt.

For example, in order to allow for the de-burring of semi-finished chromium-cobalt products, the strip consists of a support preferably made of cotton ply (very flexible), whereupon, by means of a primary resin-based adhesive, abrasive grains of friable aluminum oxide are distributed, preferably with grains of between P100 and P400 (FEPA P), with a closed coated density (surface cover > 80%), covered by a secondary resin-based adhesive and finally surface additives in order to lower the temperature within the contact area containing the semi-finished product being processed.

The grinding assembly 104 comprises a peripheral wall 128 that delimits an inner region 130, accessible for example from a side door.

The grinding assembly 104 further comprises a storage device 132, operating for example through the peripheral wall 128, adapted to be moved to transport outwards a plurality of ground semi-finished products S3 from the region 130. For example, the storage device 132 is a translatable tray.

The grinding assembly 104 further comprises an anthropomorphic robot 134, located within the region 130, in proximity to the storage device 132 for storing the ground semi-finished products S3.

The grinding assembly 104 further comprises a grinding apparatus 136 adapted to perform grinding processes on de-burred semi-finished products from the de-burring assembly 102, located within the region 130, so that the robot 134 may approach the semi-finished products to be ground to perform the grinding processes.

For example, the grinding apparatus 136 comprises a first grinding device 136a and a second grinding device 136b, arranged in front of the robot 134, transversely separated one from the other, each comprising one or more grinding wheels whereupon finishing strips are applied that are adapted to perform grinding processes on metallic semi-finished products, in particular made of chromium-cobalt.

For example, in order to allow semi-finished chromium-cobalt products to be ground, the strip consists of a support preferably made of polyester ply (heavy and with high tear resistance), whereupon, by means of a primary resin-based adhesive, abrasive grains of ceramic materials are distributed, preferably with grains of between P36 and P200 (FEPA P), with a closed coating density (surface cover > 80%), covered by a secondary resin-based adhesive and surface additives in order to lower the temperature within the contact area containing the semi-finished product being processed.

Finally, the transfer assembly 106, comprising for example a translatable tray, operates between the de-burring assembly 102 and the grinding assembly 104 and is adapted to be supplied, for example by the robot 114 of the de-burring assembly 102, with de-burred semi-finished products, being moved from the region 110 of the de-burring assembly 102 to the region 130 of the grinding assembly 104 and offering, for example to the robot 134 of the grinding assembly 104, de-burred semi-finished products that are to be ground (semi-finished products to be ground) .

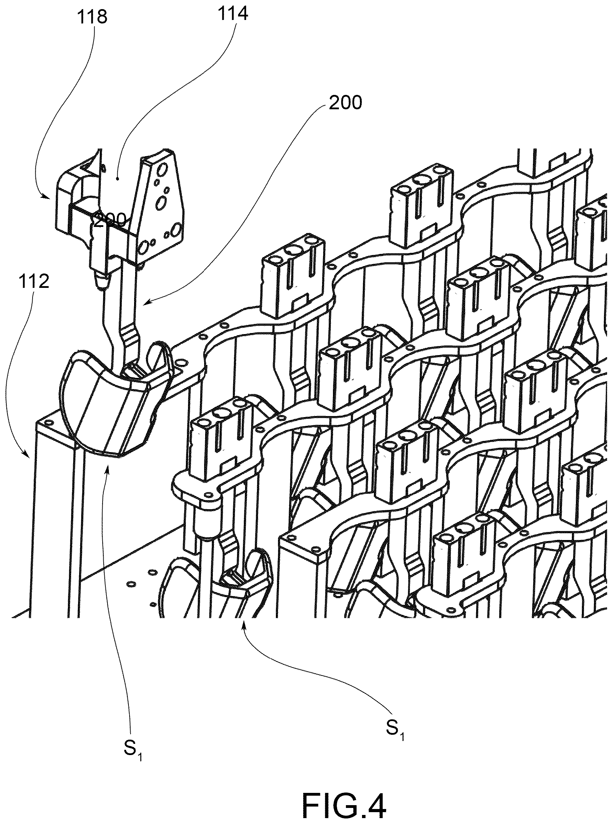

Referring to Figure 4, the feed device 112 supports a plurality of semi-finished products to be de-burred S1. Each semi-finished product to be de-burred is supported by a respective gripping support 200, adapted to be removably arranged on the feed device 112 and adapted to be gripped stably and releasably by a gripper 118 of the robot 114 of the de-burring assembly 102.

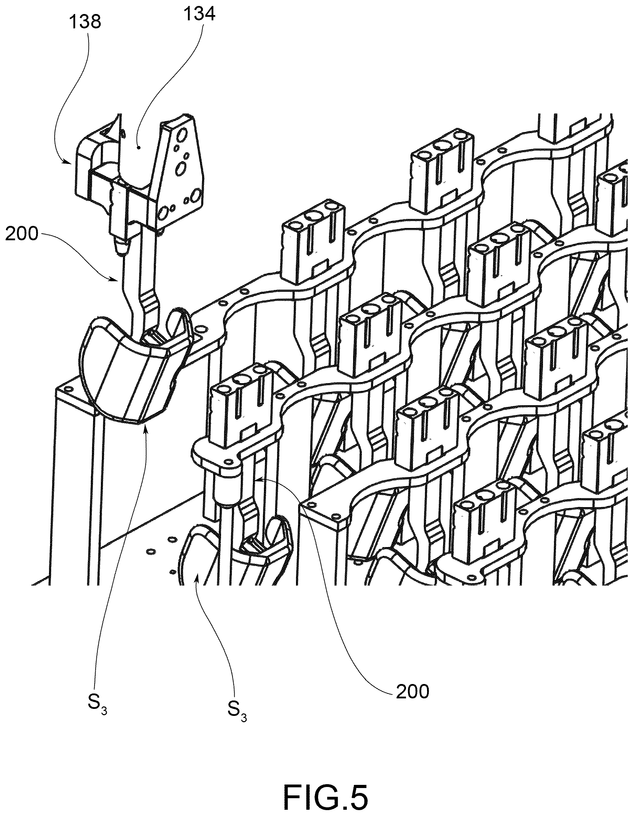

Similarly, with reference to Figure 5, the storage device 132 supports a plurality of ground semi-finished products S3. Each ground semi-finished product is supported by the respective gripping support 200, adapted to be removably arranged on the storage device 132 and adapted to be stably and releasably gripped by a gripper 138 of the robot 134 of the grinding assembly 104.

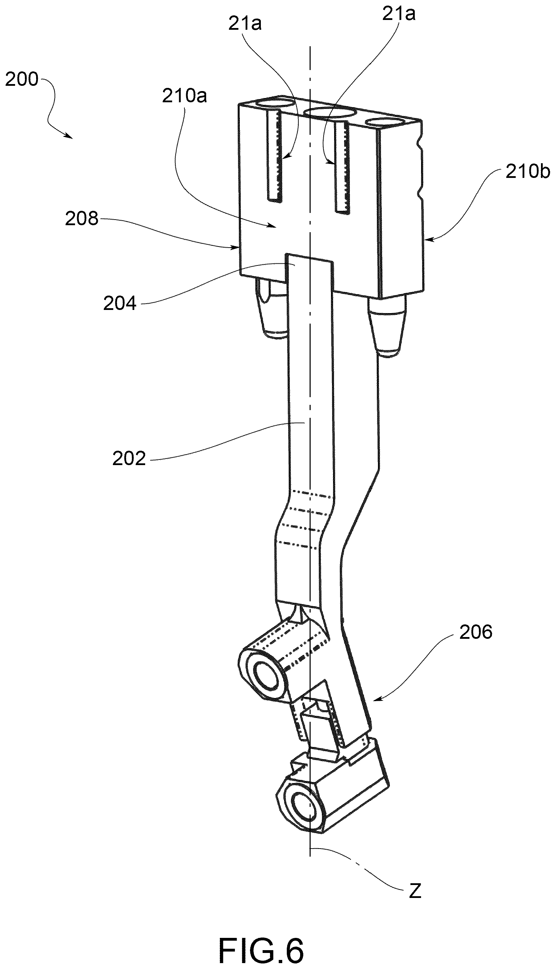

With reference to Figure 6, the gripping support 200, according to an embodiment of the present invention, is adjustable to allow stable engagement with semi-finished pieces corresponding to femoral components of different sizes.

For example, the gripping support 200 comprises a rod 202 extending along a rod axis Z between a proximal end 204, on the side intended for gripping of the gripper 118, 138 of the robot 114, 134, and a distal end 206, on the side intended for engagement with the semi-finished products S1, S3.

The gripping support 200 further comprises a gripping portion 208 applied to the proximal end 204 of the rod 202. The gripping portion 208 is a block having a front face 210a and a rear face 210b, which are preferably flat and parallel.

The gripping portion 208 comprises shape coupling means adapted to be coupled by shape coupling to the gripper 118, 138 of the robot 114, 134. For example, said coupling means comprise at least one front groove 212a on the front face 210a of the gripping portion 208 and at least one rear groove 212b on the rear face 210b of the gripping portion 208.

Preferably, there are two front grooves, transversely spaced apart and parallel, and two rear grooves, spaced apart and parallel. For example, the front groove 212a extends along a first direction, for example parallel to the rod axis Z, and the rear groove 212b extends along a second direction, orthogonal to the first direction, for example orthogonal to the rod axis Z.

Advantageously, said coupling means allow for a very rigid grip on the part of the gripper of the robot, which is essential for processing with very tight tolerances and high-quality standards.

The gripping support 200 further comprises adjustable engagement means 220 for the stable and releasable engagement to the semi-finished products S1, S3. Said engagement means 220, located at the distal end 206 of the rod 202, comprise a tang 222 of the rod 202, a first engagement body 224 protruding from the tang 222, wherein a first engagement hole 226 is obtained having a first hole axis H1 incident to the rod axis Z, and a second engagement body 228 wherein a second engagement hole 230 having a second axis for H2 parallel to the first hole axis H1 is obtained.

Said engagement holes 226, 230 are adapted to contain a respective pin 8a, 8b of the semi-finished products S1, S3 and the distance between the first hole axis H1 and the second hole axis H2 is adjustable to match the sizes of the femoral component to be machined. In particular, the tang 202 comprises a pair of prongs 232a, 232b between which a portion 234 of the second engagement body 228 is slidingly guided.

Preferably, the relative translation between the first hole axis H1 and the second hole axis H2, i.e., the relative translation between the tang 222 and the second engagement body 228, occurs along a direction of translation T, which intersects the rod axis Z.

Preferably, the gripping support 200 comprises a soft bushing 250, for example made of a plastics material, adapted to be inserted into a respective engagement hole 226, 230; the pin 8a, 8b is then housed inside the bushing. Advantageously, this avoids contamination of the semi-finished product on the part of the tang material and any damage thereto.

Said engagement means 220 comprise releasable forcing means adapted to apply a locking action to the pins 8a, 8b; in particular, the forcing means are adapted to engage the second engagement body (228) and the tang (222) to apply a locking action to the pins (8a,8b). For example, said forcing means comprise a screw 260 screwable between the tang 222 and the second engagement body 228, along the direction of translation T. Once the pins 8a, 8b are inserted into the respective engagement holes 226, 230, the screw 260 is manually screwed by an operator to move the second engagement body 228 closer to the tang 222 and thus forcibly lock the semi-finished product to the gripping support due to the frictional forces generated between the parts that are in contact.

Obviously, unscrewing the screw 260 releases the forcing and it is then possible to separate the semi-finished product S1, S3 from the gripping support.

Advantageously, the gripping support according to the present invention meets the needs of the industry in that it allows semi-finished products of femoral components of different sizes to be gripped effectively.

Advantageously, moreover, the robotic station according to the present invention performs both de-burring and grinding of semi-finished chromium-cobalt products.

According to the prior art, in fact, a semi-finished chromium-cobalt product produced by the casting is machined, for example milled, in order to complete a first de-burring or roughing and then processed in a grinding device to obtain the semi-finished piece. However, this results in longer processing times and lower quality of the milled surfaces, which affects the finished product.

According to the invention, however, the semi-finished product resulting from the casting is directly processed on the robotic station, first for de-burring and then for grinding.

It is clear that a person skilled in the art may make changes to the robotic station and to the gripping support described above in order to meet contingent needs, which changes all fall within the scope of protection as defined in the following claims.

## Claims

1. A gripping support (200) for a gripper (118,138) of a robot (114,134), comprising: a rod (202), extending along a rod axis (Z) between a proximal end (204) and a distal end (206), a gripping portion (208), at the proximal end (204) of the rod (202), and adjustable engagement means (220) arranged at the distal end (206) of the rod (202) to releasably engage a semi-finished product (S1, S3) of a biomedical prosthesis, wherein the engagement means (220) comprise a tang (222) of the rod (202), a first engagement hole (226) obtained in the tang (222), having a first hole axis (H1) incident to the rod axis (Z), and a second engagement body (228) having a second engagement hole (230), having a second hole axis (H2) parallel to the first hole axis (H1), said engagement holes (226, 230) being adapted to receive a respective pin (8a, 8b) of the semi-finished product (S1, S3), wherein the second engagement body (228) and the tang (222) are slidingly coupled to each other to adjust the distance between the first hole axis (H1) and the second hole axis (H2) and wherein the engagement means (220) comprise releasable forcing means adapted to engage with the second engagement body (228) and the tang (222) in order to apply a locking action to the pins (8a, 8b).

2. A gripping support according to claim 1, wherein the adjustment between the first hole axis (H1) and the second hole axis (H2) occurs along a translation direction (T) intersecting the rod axis (Z).

3. A gripping support according to claim 2, wherein the tang (222) comprises a pair of prongs (232a, 232b) between which a portion (234) of the second engagement body (228) is slidingly guided.

4. A gripping support according to any one of the preceding claims, comprising at least one bushing (250) made of a plastic material, adapted to be inserted into the respective engagement hole (226, 230), said bushing (250) being adapted to house the respective pin (8a, 8b).

5. A gripping support according to any one of the preceding claims, wherein the forcing means comprise a screw (260) screwable between the tang (222) and the second engagement body (228) to mutually move them close to or apart from each other.

6. A gripping support according to claim 5 when dependent on claim 2, wherein the screw (260) is screwable along the translation direction (T).

7. A gripping support according to any one of the preceding claims, wherein the gripping portion (208) comprises shape coupling means adapted to be coupled by shape coupling to the gripper (118, 138) of the robot (114, 134).

8. A gripping support according to claim 7, wherein the gripping portion (208) is a block having a front face (210a) and a rear face (210b) and the coupling means comprise at least one front groove (212a) on the front face (210a) and at least one rear groove (212b) on the rear face (210b).

9. A gripping support according to claim 8, wherein the front groove (212a) extends along a first direction, for example parallel to the rod axis (Z), and the rear groove (212b) extends along a second direction, orthogonal to the first direction, for example orthogonal to the rod axis (Z).

10. A robotic station (100) to perform de-burring and grinding processes on semi-finished chrome-cobalt products (S1, S3) of femoral components (1) of knee prostheses, comprising:
- a de-burring apparatus (116) adapted to perform de-burring processes on semi-finished products (S1) to be de-burred, comprising one or more de-burring wheels and one or more finishing belts applied to said wheels to perform de-burring processes on semi-finished chrome-cobalt products;
- a grinding apparatus (136) adapted to perform grinding processes on semi-finished products to be ground, comprising one or more grinding wheels and one or more finishing belts applied to said wheels to perform grinding processes on semi-finished chrome-cobalt products;
- at least one anthropomorphic robot (114, 134) comprising a gripper (118, 138) for a gripping device (200) supporting the semi-finished product (S1, S3), adapted to move the semi-finished product (S1) to the de-burring apparatus (116) and/or the semi-finished product (S3) to the grinding apparatus to perform the de-burring and grinding processes.

11. A robotic station according to claim 10, wherein the finishing belt of the de-burring apparatus has one or more of the following features: cotton sheet support, abrasive aluminum oxide grains, grain size between P100 and P400, closed coating density, surface additive.

12. A robotic station according to claim 10 or 11, wherein the finishing belt of the grinding apparatus has one or more of the following features: polyester sheet support, abrasive ceramic material grains, grain size between P36 and P200, closed coating density, surface additive.
